(19) **Europäisches Patentamt**
**European Patent Office**
**Office européen des brevets**

(11) **EP 4 117 504 B1**

(12) **EUROPEAN PATENT SPECIFICATION**

(45) Date of publication and mention
of the grant of the patent:
**01.10.2025 Bulletin 2025/40**

(21) Application number: **21715094.5**

(22) Date of filing: **09.03.2021**

(51) International Patent Classification (IPC):
**A61B 3/10** *(2006.01)*        **A61B 3/12** *(2006.01)*
**A61B 3/13** *(2006.01)*

(52) Cooperative Patent Classification (CPC):
**A61B 3/102; A61B 3/1225; A61B 3/13**

(86) International application number:
**PCT/US2021/021488**

(87) International publication number:
**WO 2021/183499 (16.09.2021 Gazette 2021/37)**

(54) **SYSTEMS, METHODS AND COMPUTER PROGRAM PRODUCTS FOR IDENTIFYING PRESENCE OF A CONJUGATE IN AN IMAGE**

SYSTEME, VERFAHREN UND COMPUTER PROGRAMM PRODUKT ZUR IDENTIFIZIERUNG EINES KONJUGATS IN EINEM BILD

SYTÈMES, PROCÉDÉS ET LOGICIELS POUR INDENTIFIER UN CONJUGUÉ DANS UNE IMAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.03.2020 US 202062987377 P**

(43) Date of publication of application:
**18.01.2023 Bulletin 2023/03**

(73) Proprietor: **Leica Microsystems NC, Inc.
Durham, NC 27703 (US)**

(72) Inventor: **HENDARGO, Hansford
Durham, North Carolina 27703 (US)**

(74) Representative: **J A Kemp LLP
80 Turnmill Street
London EC1M 5QU (GB)**

(56) References cited:
**US-A1- 2016 166 144        US-A1- 2019 049 232
US-A1- 2019 125 190        US-A1- 2020 064 117
US-A1- 2021 080 247        US-B2- 9 377 291**

- **ZAWADZKI ROBERT J ET AL: "AO-OCT with reference arm phase shifting for complex conjugate artifact-free imaging of in vivo retinal structures", OPHTHALMIC TECHNOLOGIES XXI, SPIE, 1000 20TH ST. BELLINGHAM WA 98225-6705 USA, vol. 7885, no. 1, 10 February 2011 (2011-02-10), pages 1 - 5, XP060005779, DOI: 10.1117/12.873955**
- **DAE YU KIM, JOHN S. WERNER, ROBERT J. ZAWADZKI: "Comparison of phase-shifting techniques for in vivo full-range, high-speed Fourier-domain optical coherencetomography", JOURNAL OF BIOMEDICAL OPTICS, SPIE, PO BOX 10 BELLINGHAM WA 98227-0010, USA, vol. 15, no. 5, 12 October 2010 (2010-10-12), pages 056011-1 - 056011-8, XP040544050, DOI: 10.1117/1.3494556**
- **FELIX K?TTIG ET AL: "An advanced algorithm for dispersion encoded full range frequency domain optical coherence tomography", OPTICS EXPRESS, vol. 20, no. 22, 22 October 2012 (2012-10-22), pages 24925, XP055136882, ISSN: 1094-4087, DOI: 10.1364/OE.20.024925**

EP 4 117 504 B1

**Description**

CLAIM OF PRIORITY

The present application claims priority to United States Provisional Application Serial No. 62/987,377, filed on March 10, 2020, entitled *Methods, Systems and Devices for Detecting Presence of Conjugate Images.*

FIELD

The present inventive concept relates generally to imaging and, more particularly, to optical coherence tomography (OCT) imaging systems.

BACKGROUND

Optical coherence tomography (OCT) uses Fourier processing of optical interferograms to obtain depth-resolved profiles of a sample. This processing inherently leads to a mirror image known as the complex conjugate image that may reduce the likelihood of, or possibly prevent, accurate visualization of the desired image. Without complicated hardware or other sources of information, separating the conjugate image from the real image may present a great challenge.

Conventional methods have been developed to reduce, or possibly eliminate, the complex conjugate image. However, these conventional methods often require complex hardware, multiple image acquisitions, complex software processing, and/or prior knowledge of physical composition of the sample being imaged. Conventional methods are discussed, for example, in United States Patent Numbers 8,414,564 and 7,336,366B2.

US 2016166144 A1, titled "Methods for Comprehensive Fourier Domain Optical Coherence Tomography (FDOCT)", discusses optical coherence tomography systems that can capture images of the entire eye. These systems have a sample arm with focal optics that can switch between at least two scanning modes in less than 1.0 second.

Kim et al: "Comparison of phase-shifting techniques for in vivo full-range, high-speed Fourier-domain optical coherence tomography", discusses advantages and disadvantages of different phase-shifting techniques for use in vivo full-range, high-speed Fourier-domain OCT.

In Zawadzki et al: "AO-OCT with reference arm phase shifting for complex conjugate artifact-free imaging of in vivo retinal structures", the performance of an AO-OCT system with reference arm phase shifting for complex conjugate artifactfree imaging of in vivo retinal structures is discussed. As a complex conjugate artifact removal (CCR) method, a technique requiring constant phase shifts between consecutive A-scans was used, with the shifts being generated by continuous beam path-length changes from offsetting the pivot point of the scanning mirror placed in the system reference arm. In order to reconstruct the complex spectral fringe pattern, Fourier transformation along the transverse axis and a filtering algorithm are used.

SUMMARY

Some embodiments of the present inventive concept provide a system for identifying presence of a conjugate in an image comprising one or more processors and one or more storage devices. The system is configured to acquire spectral data associated with a sample to be imaged. Dispersion coefficients are optimized for acquired spectral data. A corrective phase function is calculated using the optimized dispersion coefficients. A negative corrective phase function is applied to a signal to provide a resulting image. It is determined if the resulting image has degraded signal strength or an enhanced signal strength relative to an original image. A reference arm shift is calculated if it is determined that the resulting image has enhanced signal strength. A position of a reference arm of the system is adjusted based on the calculated reference arm shift to move a conjugate image out of view.

In further embodiments, the degraded signal strength may indicate a non-conjugate signal and an enhanced signal strength may indicates a conjugate signal.

In still further embodiments, the spectral data may include one of a whole image of the sample, a plurality of A-scans of the sample, data from a plurality of A-scans and sub-regions of the image including a portion of data from a plurality of A-scans .

In some embodiments, the resulting image may be an image produced after application of a fast Fourier transform (FFT).

In further embodiments, the system may be further configured to adjust the position of the reference arm manually or automatically.

In still further embodiments, the system may be further configured to calculate the corrective phase function $\phi_c(k)$ using the following equation:

$$\phi_c(k) = c_1(k - k_o)^2 + c_2(k - k_o)^3,$$

where $c_1$ is a first correction coefficient, $c_2$ is a second correction coefficient, $k$ is a source wavenumber and $k_o$ is a center wavenumber of a source.

In some embodiments, the system may be configured to adjust the position of the reference arm by providing feedback to the reference arm that causes the reference arm to move a reference reflector to a position where an upright image is in view and a conjugate image is hidden.

In further embodiments, the system may be an optical coherence tomography (OCT) imaging system.

In still further embodiments, the system may further include a microscope.

Some embodiments of the present inventive concept provide a method for identifying presence of a conjugate in an image in a system comprising one or more processors and one or more storage devices. The method includes acquiring spectral data associated with a sample to be imaged; optimizing dispersion coefficients for acquired spectral data; calculating a corrective phase function using the optimized dispersion coefficients; applying a negative corrective phase function to a signal to provide a resulting image; determining if the resulting image has degraded signal strength or an enhanced signal strength relative to an original image; calculating a reference arm shift if it is determined that the resulting image has enhanced signal strength; and adjusting a position of a reference arm of the system based on the calculated reference arm shift to move a conjugate image out of view.

Related computer program products are also provided.

BRIEF DESCRIPTION OF THE DRAWINGS

Fig. 1 is a simple block diagram illustrating a Fourier domain optical coherence tomography (FD-OCT) system.

Fig. 2 is a block diagram illustrating an example optical coherency tomography (OCT) retinal (posterior) imaging system.

Fig. 3 is a block diagram illustrating an example OCT cornea (anterior) imaging system.

Figs. 4A and 4B are graphs illustrating the magnitude of the Fourier transforms of a real signal affected by dispersion over positive (+f) and negative (-f) frequencies in accordance with embodiments of the present inventive concept.

Figs. 5A and 5B are images of both positive (upright) image and conjugate (upside down) image produced by an FD-OCT imaging system in accordance with some embodiments of the present inventive concept.

Fig. 6 illustrates an OCT image of human cornea with overlapping conjugate which may prevent an accurate visualization of the desired upright image even if only the lower half of the image is shown.

Figs. 7 and 8 are flowcharts illustrating operations for conjugate evasion in accordance with various embodiments of the present inventive concept.

Fig. 9 is a block diagram of a data processing system in communication with an imaging system that may be used to implement processes in accordance with various embodiments of the present inventive concept.

Fig. 10 is block diagram a system in accordance with some embodiments of the present inventive concept including a microscope.

DETAILED DESCRIPTION

The present inventive concept will be described more fully hereinafter with reference to the accompanying figures, in which embodiments of the inventive concept are shown. This inventive concept may, however, be embodied in many alternate forms and should not be construed as limited to the embodiments set forth herein.

Accordingly, while the inventive concept is susceptible to various modifications and alternative forms, specific embodiments thereof are shown by way of example in the drawings and will herein be described in detail. It should be understood, however, that there is no intent to limit the inventive concept to the particular forms disclosed, but on the contrary, the inventive concept is to cover all modifications, equivalents, and alternatives as defined by the claims. Like numbers refer to like elements throughout the description of the figures.

The terminology used herein is for the purpose of describing particular embodiments only and is not intended to be limiting of the inventive concept. As used herein, the singular forms "a", "an" and "the" are intended to include the plural forms as well, unless the context clearly indicates otherwise. It will be further understood that the terms "comprises", "comprising," "includes" and/or "including" when used in this specification, specify the presence of stated features, integers, steps, operations, elements, and/or components, but do not preclude the presence or addition of one or more other features, integers, steps, operations, elements, components, and/or groups thereof. Moreover, when an element is referred to as being "responsive" or "connected" to another element, it can be directly responsive or connected to the other element, or intervening elements may be present. In contrast, when an element is referred to as being "directly responsive" or "directly connected" to another element, there are no intervening elements present. As used herein the term "and/or" includes any and all combinations of one or more of the associated listed items and may be abbreviated as "/".

Unless otherwise defined, all terms (including technical and scientific terms) used herein have the same meaning as commonly understood by one of ordinary skill in the art to which this inventive concept belongs. It will be further understood that terms used herein should be interpreted as having a meaning that is consistent with their meaning in the context of this specification and the relevant art and will not be interpreted in an idealized or overly formal sense unless expressly so defined herein.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

It will be understood that, although the terms first, second, etc. may be used herein to describe various elements, these elements should not be limited by these terms. These terms are only used to distinguish one element from another. For example, a first element could be termed a second element, and, similarly, a second element could be termed a first element without departing from the teachings of the disclosure. Although some of the diagrams include arrows on communication paths to show a primary direction of communication, it is to be understood that communication may occur in the opposite direction to the depicted arrows.

Aspects of the present disclosure are described herein with reference to flowchart illustrations and/or block diagrams of methods, apparatuses (systems) and computer program products according to embodiments of the disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable instruction execution apparatus, create a mechanism for implementing the functions/acts specified in the flowchart and/or block diagram block or blocks. As used herein, "a processor" may refer to one or more processors.

These computer program instructions may also be stored in a computer readable medium that when executed can direct a computer, other programmable data processing apparatus, or other devices to function in a particular manner, such that the instructions when stored in the computer readable medium produce an article of manufacture including instructions which when executed, cause a computer to implement the function/act specified in the flowchart and/or block diagram block or blocks. The computer program instructions may also be loaded onto a computer, other programmable instruction execution apparatus, or other devices to cause a series of operational steps to be performed on the computer, other programmable apparatuses or other devices to produce a computer implemented process such that the instructions which execute on the computer or other programmable apparatus provide processes for implementing the functions/acts specified in the flowchart and/or block diagram block or blocks.

Although many of the examples discussed herein refer to the sample/subject being an eye, specifically, the retina, cornea, anterior segment and lens of the eye, embodiments of the present inventive concept are not limited to this type of sample. Any type of sample that may be used in conjunction with embodiments discussed herein may be used without departing from the scope of the present inventive concept.

Although embodiments of the present inventive concept focus on the use of OCT to scan the sample, embodiments of the present inventive concept are not limited to the use of OCT. It will be understood that any method and system used to scan a sample can be used without departing from the scope of the present inventive concept.

As used herein, "subject" refers to a person or thing or a portion of a person or thing being imaged. It will be understood that although embodiments of the present inventive concept are discussed herein with respect to an eye being the subject, embodiments of the present inventive concept are not limited to this configuration. The subject can be any subject, including, for example, veterinary, cadaver study or human subject without departing from the scope of the present inventive concept.

As discussed above, optical coherence tomography (OCT) uses Fourier processing of optical interferograms to obtain depth-resolved profiles of a sample. This processing inherently leads to a mirror image known as the "complex conjugate image" that may reduce the likelihood of, or possibly prevent, accurate visualization of the desired image. Without complicated hardware or other sources of information, separating the conjugate image from the real image is a great challenge. Conventional methods have been developed to reduce or possibly eliminate the complex conjugate image. However, these conventional methods often require complex hardware, multiple image acquisitions, complex software processing, and/or prior knowledge of physical composition of the sample being imaged. Accordingly, embodiments of the present inventive concept provide methods for reducing the effect of the complex conjugate for arbitrary samples that may be compatible with standard OCT systems.

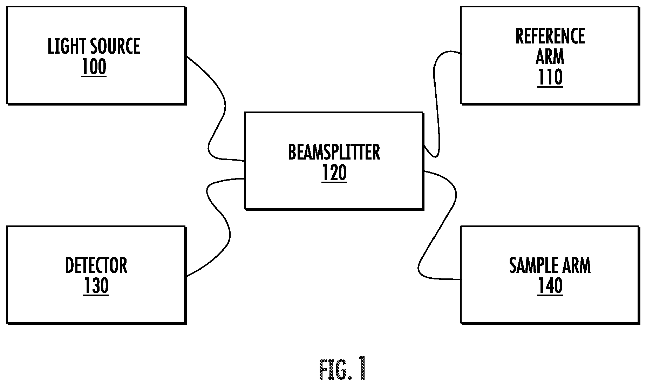

In particular, embodiments of the present inventive concept provide methods for detecting if the complex conjugate image is present in a given image and for moving the complex conjugate out of view by adjusting the reference arm reflector position if the complex conjugate image is present. Conventionally, it is very difficult to identify if the complex conjugate image is even present in the image. As will be discussed below, the complex conjugate image may be detected by processing a portion of the original image having different dispersion parameters. If the complex conjugate image is detected, the reference arm position of the OCT system is adjusted to move the complex conjugate image out of view and bring the desired upright image into view. Methods in accordance with embodiments of the present inventive concept may be implemented with existing OCT systems and, thus, no modification to existing hardware may be required. Details of the present inventive concept will be discussed below with respect to Figs. 1 through 10.

Referring first to Fig. 1, a simple block diagram of an example Fourier domain OCT (FD-OCT) imaging system will be discussed. FD-OCT imaging systems use the principles of low-coherence interferometry to generate three dimensional (3D) images of a sample. As illustrated in Fig. 1, the FD-OCT system generally includes a light source 100, a detector 130 (detection), a beamsplitter 120, a reference arm 110, and a sample arm 140.

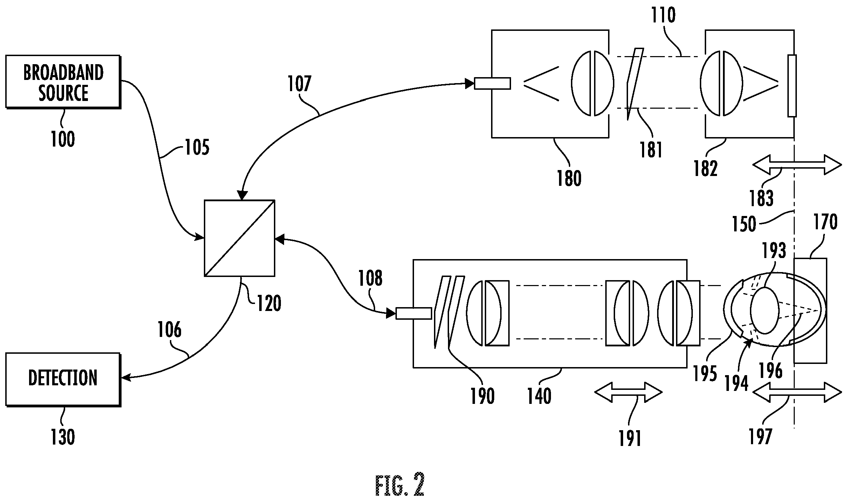

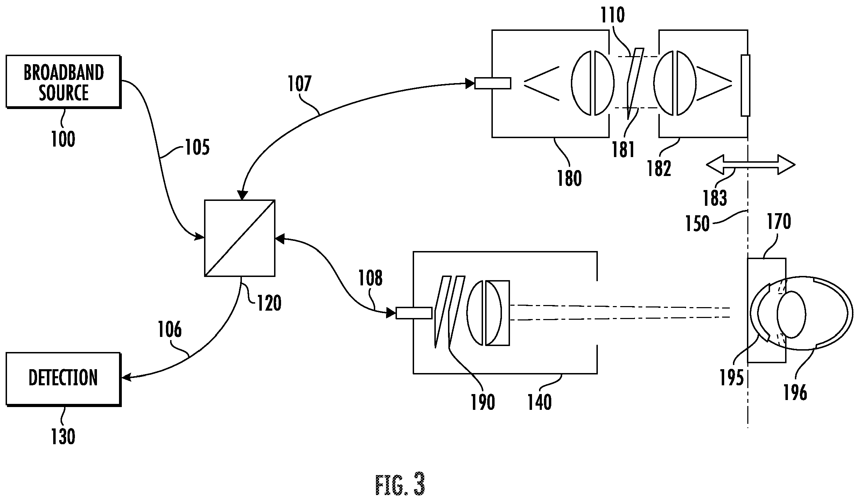

More detailed block diagrams of FD-OCT systems that may be used in accordance with embodiments of the present inventive concept will now be discussed with respect to Figs. 2 and 3. It will be understood that the systems in Figs. 1 through 3 are provided for example purposes only and, thus, embodiments of the present inventive concept should not be limited thereto. Fig. 2 is a block diagram an FD-OCT retinal imaging system. As illustrated in Fig. 2, the system includes a broadband source 100, a reference arm 110 and a sample arm 140 coupled to each other by a beamsplitter 120. The beamsplitter 120 may be, for example, a fiber optic coupler or a bulk or micro-optic coupler. The beamsplitter 120 may provide from about a 50/50 to about a 90/10 split ratio. As further illustrated in Fig. 2, the beamsplitter 120 is also coupled to a wavelength or frequency sampled detection module 130 over a detection path 106 that may be provided by an optical fiber.

As further illustrated in Fig. 2, the source 100 is coupled to the beamsplitter 120 by a source path 105. The source 100 may be, for example, a continuous wave broadband superluminescent diode, a pulsed broadband source, or tunable source. The reference arm 110 is coupled to the beamsplitter 120 over a reference arm path 107. Similarly, the sample arm 140 is coupled to the beamsplitter 120 over the sample arm path 108. The source path 105, the reference arm path 107 and the sample arm path 108 may all be provided by optical fiber or a combination of optical fiber, free-space, and bulk- or micro-optical elements.

As illustrated in Fig. 2, the reference arm of the FD-OCT retinal imaging system may include a collimator assembly 180, a variable attenuator 181 that may include a neutral density filter or a variable aperture, a mirror assembly 182, a reference arm variable path length adjustment 183 and a path length matching position 150, *i.e.* optical path length matching between the reference arm path length and the sample arm path length to the subject region of interest. As further illustrated, the sample arm 140 may include a dual-axis scanner assembly 190 and an objective lens with variable focus 191.

The sample illustrated in Fig. 2 is an eye including a cornea 195, iris/pupil 194, ocular lens 193 and retina 196. A representation of an FD-OCT imaging window 170 is illustrated near the retina 196. The retinal imaging system relies on the objective lens plus the optics of the subject eye, notably cornea 195 and ocular lens 193, to image the posterior structures of the eye. As further illustrated the region of interest 170 within the subject is selected through coordination of the focal position 196 and reference arm path length adjustment 183, such that the path length matching position 197 within the subject is at the desired location.

Referring now to Fig. 3, a block diagram illustrating a FD-OCT corneal (anterior) imaging system will be discussed. As illustrated therein, the system of Fig. 3 is very similar to the system of Fig. 3. However, the objective lens variable focus need not be included, and is not included in Fig. 3. The anterior imaging system of Fig. 3 images the anterior structures directly, without reliance on the optics of the subject to focus on the anterior structures.

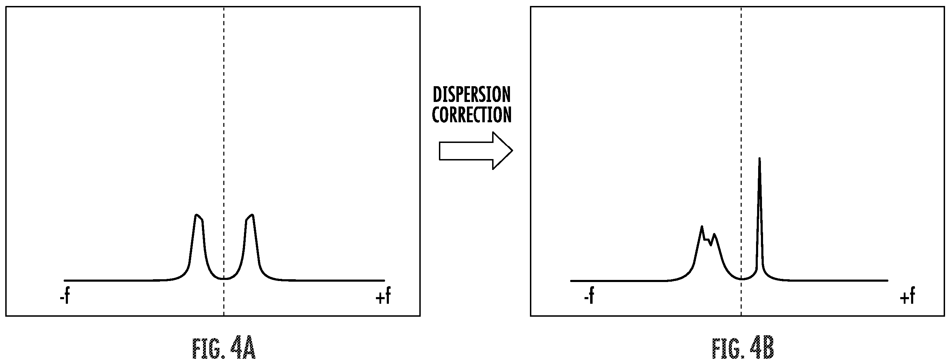

OCT processing requires taking the Fourier transform of a real-valued spectral interferogram signal, also called an A-Scan. This results in a transformed signal that contains mirrored positive and negative frequency components. These components correspond to a positive or negative displacement of the optical path length between sample reflectors and the reference reflection as shown in Figs. 4A and 4B. OCT systems also typically have a mismatch in the optics used in the sample and reference arms. This mismatch of optics may also be caused by the type of sample being imaged. These differences may cause an effect known as "dispersion" that can result in an apparent blurring of the image. Dispersive effects may be compensated by using a numerical correction which involves applying second and third order phase terms (or higher order terms as well) to the acquired interferometric spectrum. Doing so leads to a strengthening of the signal of the upright image compared to the conjugate signal as illustrated in Fig. 4B. The conjugate signal may be corrected by application of the same numerical correction, but with opposite signed terms, which would then lead to a blurring of the upright signal.

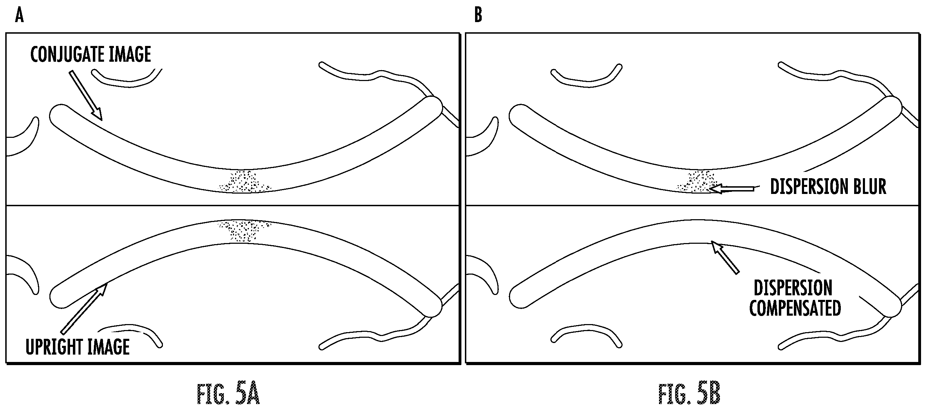

Fig. 4A illustrates that a Fourier transform of a real signal affected by dispersion yields mirrored blurred peaks over positive and negative frequencies, +f and -f. Correcting for the effects of dispersion causes the positive frequency peak (+f in Fig. 4B) to become sharper while the negative frequency peak (complex conjugate image -f in Fig. 4B) broadens in width and has a reduced height as the -f peak in Fig. 4A. Alternatively, correcting the dispersion in the negative frequencies would cause the positive frequency signal to broaden. A two-dimensional OCT image, referred to as a B-Scan, consists of multiple A-Scans. When viewing an OCT image, typically only the signal range that corresponds to frequencies on one side are viewed, *i.e.,* positive or negative frequencies, and the remaining or opposite frequencies (complex conjugate signal) are typically cropped from view. Fig. 5A illustrates a positive image (upright image) and the corresponding conjugate image (upside down image). Fig. 5B specifically illustrates the two images after dispersion correction. In particular, Figs. 5A and 5B illustrate an example of an OCT image of the human cornea showing upright and conjugate images (Fig. 5A) before dispersion correction and (Fig. 5B) after dispersion correction. As is clear from the images, the upright image is sharper after dispersion correction while the conjugate remains blurred. Typically, only the upright image would be displayed by simply cropping the top half of the image, *i.e.* only the bottom portion of the Fig. 5B would be displayed.

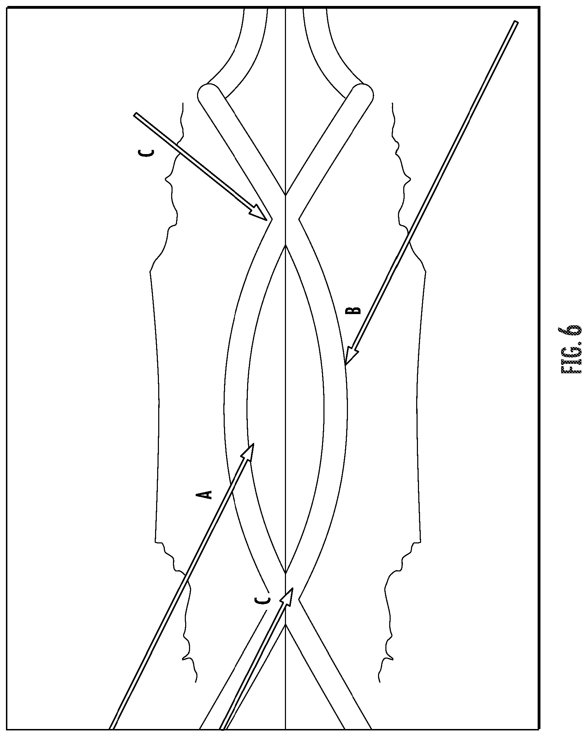

It is possible during imaging that components from one side may shift to the other due to movement of the sample or the reference arm reflector. As shown in Fig. 6, when this occurs, components from the complex conjugate signal (top signal A) may overlap (overlapping at C) the desired upright signal components (bottom signal B), thus, providing an inaccurate image of the sample. When the two signals A and B overlap at C as illustrated in Fig. 6, the imaging range of the OCT system may be limited as the complex conjugate occupies half the range and the viewing range is restricted to avoid showing the conjugate image. There is no simple way to clearly distinguish the complex conjugate signal from the upright signal without significant changes and additions to the OCT system.

Conventional techniques for suppressing or eliminating the complex conjugate artifact having been developed. However, these methods require much more complex hardware along with the acquisition of multiple images to generate a single complex conjugate free image. These conventional techniques may also increase cost, require increased imaging time, and may be more difficult to use for patient imaging due to motion artifacts and stability issues.

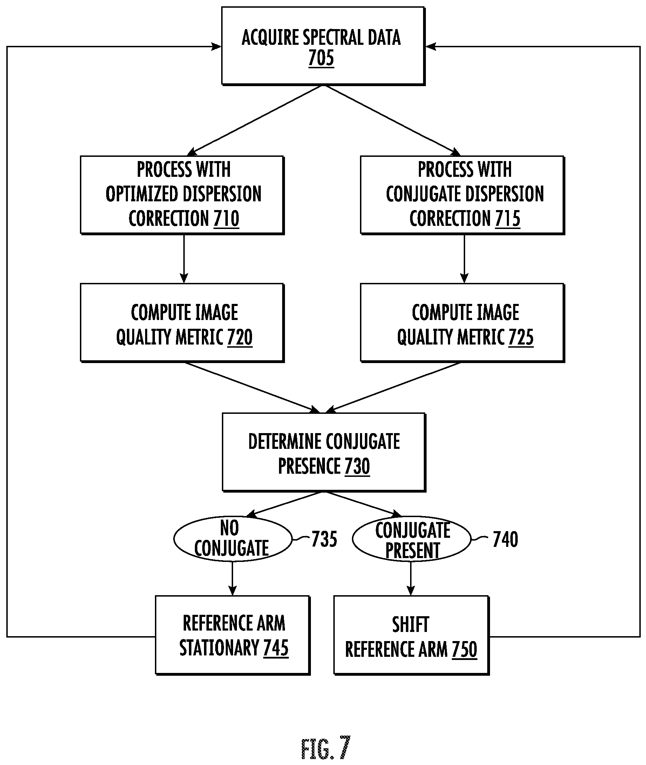

Accordingly, some embodiments of the present inventive concept provide methods for determining presence of a complex conjugate signal within a given image and reducing the likelihood that the presence thereof will obscure the desired OCT image. Fig. 7 is a flowchart illustrating operations of methods in accordance with various embodiments of the present inventive concept. As illustrated in Fig. 7, operations begin at block 705 by acquiring the raw spectral interferometric data associated with a sample to be imaged using the OCT imaging system. For example, imaging systems illustrated in Figs. 2 and 3 discussed above. Once the spectral data is acquired it may be processed in two different ways, either serially or in parallel. In particular, the spectral data may be processed with optimized dispersion correction (block 710) and the spectral data may be processed with conjugate dispersion correction (block 715). Dispersion coefficients are first optimized for a reference upright image. Conjugate dispersion coefficients have the opposite sign of the optimized coefficients. A comparison is performed using only one of the positive or negative frequency components after the Fourier transform is performed between the resulting image using the optimized dispersion coefficients and the result using the conjugate dispersion coefficients (blocks 720 and 725). It is determined if the conjugate is present (block 730). In particular, if the image resulting from the opposite signed dispersion coefficients (conjugate) has a stronger signal (block 730), then the original image can be marked as containing components from the conjugate image (block 740). If the image contains components from the conjugate (block 730), the reference arm may be shifted (block 750) responsive to the presence of the conjugate image to remove the conjugate image from view. For example, a feedback system to the reference arm can move the reference reflector to a position where the upright image is in view and the conjugate image is hidden. If, on the other hand, it is determined that no conjugate is present (block 735), the reference arm may remain stationary (block 745) as no adjustment may be needed. It will be understood that methods in accordance with those illustrated in Fig. 7 may not require additional hardware beyond the basic OCT instrumentation and can operate quickly on real-time data to actively keep the conjugate image out of view.

A more detailed description of the methods in accordance with embodiments of the present inventive concept will now be discussed. In OCT, a reflective sample target that is closely pathlength-matched to the reference arm optical path generates an interference pattern at the detector with a cross-correlation term that has the following form:

$$s(k) = \rho(k)\sqrt{r_R(k)r_S(k)}\cos[kn\Delta z_S + \phi_d(k)], \qquad \text{Eqn. (1)}$$

where $s(k)$ is the interference term of interest in the acquired spectral signal as a function of source wavenumber $k$; $\rho(k)$ is the source spectral density; $r_R(k)$ is the reflectivity of the reference reflector; $r_S(k)$ is the reflectivity of the sample reflector; $n$ is the index of refraction of the sample target; $\Delta z_S$ is the pathlength difference between the sample and reference reflectors; and $\phi_d(k)$ is a phase shift caused by dispersion. Having multiple reflectors in the sample results in a summation of Eqn. (1) over the interference signal created from each reflector.

The phase term $\phi_d$ is responsible for the broadening effect of dispersion that reduces axial resolution of the OCT image. Re-writing Eqn. (1) using Euler's formula results in:

$$s(k) = \rho(k)\sqrt{r_R(k)r_S(k)}\frac{e^{ikn\Delta z_S}e^{i\phi_d(k)}+e^{-ikn\Delta z_S}e^{-i\phi_d(k)}}{2}, \qquad \text{Eqn. (2).}$$

Taking a Fourier transform of a real-valued signal results in a signal that has mirrored displacements about the zero-frequency position as can be seen from the conjugate pair of exponentials in Eqn. (2). A corrective phase function $\phi_c(k)$ may have the form:

$$\phi_c(k) = c_1(k - k_o)^2 + c_2(k - k_o)^3, \qquad \text{Eqn. (3)}$$

where $c_1$ and $c_2$ are correction coefficients and $k_o$ is the center wavenumber of the source. The correction coefficients may be determined such that $\phi_c(k) \approx \phi_d(k)$ and can be empirically computed for the given target sample using iterative numerical optimization. It will be understood that Eqn. (3) illustrates an embodiment of the corrective phase function, but embodiments discussed herein are not limited thereto. The corrective phase function can be any polynomial with distinct coefficients.

Applying Eqn. (3) to correct for dispersion in Eqn. (2) yields:

$$s(k)e^{-i\phi_c(k)} = \rho(k)\sqrt{r_R(k)r_S(k)}\frac{e^{ikn\Delta z_S}e^{i\phi_d(k)}+e^{-ikn\Delta z_S}e^{-i\phi_d(k)}}{2}e^{-i\phi_c(k)}, \qquad \text{Eqn. (4)}$$

Thus, if $\phi_c(k) \approx \phi_d(k)$, this results in:

$$s(k)e^{-i\phi_c(k)} = \rho(k)\sqrt{r_R(k)r_S(k)}\frac{e^{ikn\Delta z_S}+e^{-ikn\Delta z_S}e^{-i2\phi_d(k)}}{2}, \qquad \text{Eqn. (5)}$$

It can be seen that the dispersive phase factor is essentially eliminated in one term (positive frequency term in this case) while the conjugate term (negative frequency term) is affected by twice the amount of dispersion. Typically, OCT images are displayed such that only either the positive or negative frequencies after the Fourier transform are visible so that the doubly dispersed signal is not seen. However, for samples that have a large extent in depth or for dynamically moving samples, it is possible that the conjugate image will have components that appear across the zero-frequency line and become visible and prevent or hinder visualization of the desired sample. Conventionally, there is no way to distinguish whether the content of the image originates from the upright or conjugate components of the signal.

Complex methods may be used to either eliminate or suppress the conjugate term but are difficult to implement in practice. Some embodiments of the present inventive concept use methods and systems discussed herein to ensure that the conjugate term remains out of the desired viewable frequencies. In particular, from Eqn. (4), it can be seen that if the corrective phase function $\phi_c$ factor is applied with the opposite sign, the negative frequency term would no longer be affected by dispersive phase while the positive frequency term would be doubly affected. From Figs. 4A and 4B, it can be seen that the result of dispersion is a broadening of the signal peak after Fourier transform as well as a reduction in signal strength. By applying negative corrective phase function $\phi_c$ to the signal, the resulting image after the Fourier transform will have its signal strength degraded if it is the positive frequency signal or enhanced if it is the conjugate signal. This enables the ability to detect whether the signal currently in view is the upright or conjugate image. If the conjugate image is detected, the reference arm position can be adjusted to move the conjugate image out of view.

Given that signal from the target of interest is present in the positive frequencies after the FFT of the spectral interferogram, the steps for determining the $\phi_c$ and detecting the conjugate will be discussed. As illustrated therein, methods for determining the corrective phase function begins at block 800 by determining the corrective phase function $\phi_c$ for the positive frequencies by using an optimization algorithm, such as Nelder-Mead or other similar methods. A metric is used to measure the quality of the image, such as mean or peak signal to noise ratio (SNR) or image sharpness, and the coefficients $c_1$ and $c_2$ (Eqn. (3) are adjusted until the optimization finds the best quality image. For example:

$$\widehat{S}(z) = FFT(s(k)e^{-i\phi_c(k)}), \qquad \text{Eqn. (6)}$$

$$\max_{\substack{c_1,c_2 \in \mathbb{R}, \\ z>0}}\left[M\{|\widehat{S}(z)|\}\right], \qquad \text{Eqn. (7)}$$

where *FFT* is the fast Fourier transform (FFT), and $\hat{S}(z)$ indicates the resulting domain change after the Fourier transform.

Over the positive frequencies (z > 0), the optimal dispersion correction coefficients, $c_1$ and $c_2$, yield the OCT image with the strongest image quality function, M, which is based on the magnitude of S(z). *M* can be any suitable image quality metric. Once the coefficients, $c_1$ and $c_2$, are determined for a given sample, they generally do not need to be recomputed unless the sample or optical medium changes.

For every acquired image, in addition to applying $\phi_c$ as in Eqn. (4), a separate, parallel processing routine using $-\phi_c$ is performed. In particular:

$$\widehat{S}_+(z) = FFT\big(s(k)e^{-i\phi_c(k)}\big) \qquad \text{Eqn. (8)}$$

$$\widehat{S}_-(z) = FFT\big(s(k)e^{i\phi_c(k)}\big) \qquad \text{Eqn. (9)}$$

where $\hat{S}_+(z)$ is the result of the FFT using $\phi_c$ and $\hat{S}_-(z)$ is the result of the FFT using $-\phi_c$. The magnitude image generated from using $-\phi_c$ is compared to the image generated from $\phi_c$ using the image quality metric discussed above. If the image quality metric degrades when using $-\phi_c$, it can be inferred that the upright image is present. If the image quality metric improves when using $-\phi_c$, this indicates that the conjugate image is in view and a shift of the reference arm is needed to move the upright image back into view and the conjugate image out of view. In particular:

$$C(\hat{S}(z)) = \begin{cases} M\big\{|\hat{S}_+(z)|\big\} \geq M\big\{|\hat{S}_-(z)|\big\}, False \\ M\big\{|\hat{S}_+(z)|\big\} < M\big\{|\hat{S}_-(z)|\big\}, True \end{cases}, \qquad \text{Eqn. (10)}$$

where *C* is a Boolean function describing whether the image of S(z) has significant conjugate signal. It some embodiments, this processing may be done on a partial image, *i.e.*, a subset of A-scans from the given frame, to reduce computation time.

Furthermore, it is possible that for samples with a large extent in depth, such as the anterior segment of the eye, that portions of the conjugate image may be visible along with portions of the upright image. In these embodiments, the window range over z may be defined to analyse specific portions of the image for the presence of conjugate signal.

If the conjugate is detected, then the reference arm position can be adjusted to move the conjugate image out of view and maintain the view on the upright image. Decision logic, such as thresholding, can be used to determine whether the reference arm should be shifted based on the extent of the difference between the metrics computed for the positive and negative dispersion corrected images. Further computations can be performed to determine the position of the conjugate and the corresponding distance the reference arm would need to move for proper adjustment. Methods, such as a center-of-mass computation, can yield the position of the overall signal in the frame and give an estimate for how far the reference arm needs to move to shift the conjugate image out of view. Other methods involving machine learning techniques can also be used to detect the presence of the sample target and even specific features within the sample, for example, a cornea or a lens in the anterior segment of the eye.

Some embodiments of the present inventive concept provide access to different sub-regions or sub-samples of the image to determine more specifically which portions of the image contain the conjugate artifact and to speed up processing times. An assessment of the extent to which the conjugate image occupies each of the sub-regions can be made to determine whether the reference arm must be shifted. In some embodiments, the conjugate artifact may directly overlap the signal of interest. In these embodiments, application of either the original or conjugate dispersion parameters may yield a similar signal. This result can also yield information on areas of the image where the conjugate is overlapping and may need to be shifted. In some embodiments, additional dispersion elements may be introduced into the optical hardware subsystems to induce a greater dispersive effect to enhance the difference between the conjugate and upright images.

It will be understood that if an adjustment of the reference arm is necessary, this adjustment may be made manually or automatically without departing from the scope of the present inventive concept. In other words, some embodiments of the present inventive concept involve automatically adjusting the position of the reference arm so that only the desired image is in view.

Although specific examples of identifying the presence of the conjugate image are discussed above, embodiments of the present inventive concept are not limited thereto. For example, in some embodiments other signal properties may be used to distinguish the conjugate components. In further embodiments the image may be compared to a reference image to determine the presence of a conjugate.

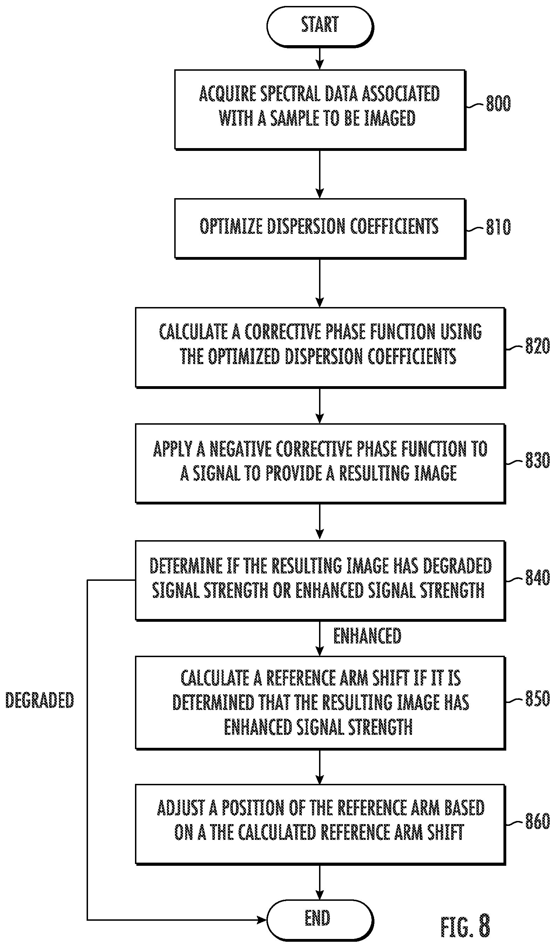

Referring now to the flowchart of Fig. 8, operations for identifying presence of a conjugate in an image in accordance with some embodiments of the present inventive concept will be discussed. As illustrated, operations begin at block 800 by acquiring spectral data associated with a sample to be imaged. The spectral data may be acquired using an OCT imaging system, for example, systems illustrated in Fig. 1 and 2, in some embodiments. The dispersion coefficients

EP 4 117 504 B1

for acquired spectral data may be optimized as discussed above (block 810). As discussed, once the coefficients, $c_1$ and $c_2$, are determined for a given sample, they generally do not need to be recomputed unless the sample or optical medium changes. A corrective phase function is calculated using the optimized dispersion coefficients (block 820). For example, the corrective phase function may be calculated using Eqn. (3) set out above. A negative corrective phase function may be applied to a signal to provide a resulting image (block 830). It is determined if the resulting image has degraded signal strength or an enhanced signal strength relative to an original image (block 840). If it is determined that the resulting image has degraded signal strength (block 840), it is concluded that the non-conjugate image (upright image) is present, then operations proceed to the end as no adjustment in the reference arm is needed.

If, on the other hand, it is determined that the resulting image has an enhanced signal strength (block 840), a "reference arm shift" is calculated (block 850). As used herein "a reference arm shift" refers to a representation of how much the reference arm should be adjusted when it is determined that the signal is enhanced and, therefore, the conjugate image is present. A position of a reference arm of the system is adjusted based on the calculated reference arm shift to move a conjugate image out of view (block 860). The adjustment may be either manually or automatically responsive to detection of the conjugate signal without departing from the scope of the present inventive concept.

Thus, a degraded signal strength indicates a non-conjugate signal (upright) and an enhanced signal strength indicates a conjugate signal. Thus, when the conjugate signal is present, the reference arm is adjusted to move the conjugate signal out of view. Thus, feedback may be provided to the reference arm that causes the reference arm to move a reference reflector to a position where an upright image is in view and a conjugate image is hidden.

It will be understood, in some embodiments, the entire image may not be needed to determine if the conjugate signal is present. Thus, in some embodiments, the determination in block 840 may be made using a plurality of A-scans of the sample that is less than the entire image. The determination may also be made using a whole image of the sample, a plurality of A-scans of the sample, data from a plurality of A-scans or sub-regions of the image including a portion of data from a plurality of A-scans.

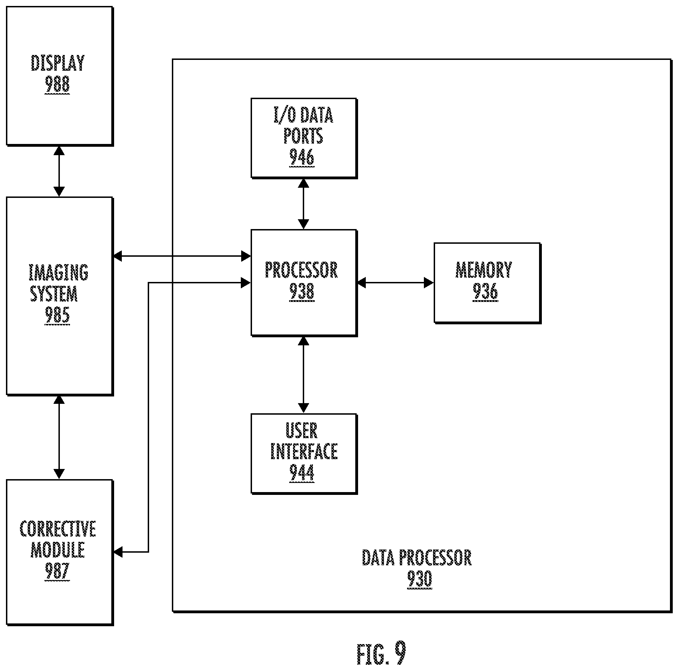

As is clear from the discussion of embodiments of the present inventive concept above, many of the methods discussed herein require processing provided by a computing device. Referring now to Fig. 9, example embodiments of a data processing system 930 in communication with an imaging system 985 configured in accordance with embodiments of the present inventive concept will be discussed with respect to Fig. 9. As will be understood, the data processing system may be included in the imaging system 985 of, for example, Figs. 2 and 3, or may be a separate device that communications with the system in Figs. 2 and 3 without departing from the scope of the present inventive concept. As further illustrated, the data processing system 930 communicates with a display 988 to display the images as well as the corrective module 987 that implements operations in accordance with embodiments discussed above. The data processing system 930 may include a user interface 944, including, for example, input device(s) such as a keyboard or keypad, a display, a speaker and/or microphone, and a memory 936 that communicate with a processor 938. The data processing system 930 may further include I/O data port(s) 946 that also communicates with the processor 938. The I/O data ports 946 can be used to transfer information between the data processing system 930 and another computer system or a network using, for example, an Internet Protocol (IP) connection. These components may be conventional components such as those used in many conventional data processing systems, which may be configured to operate as described herein.

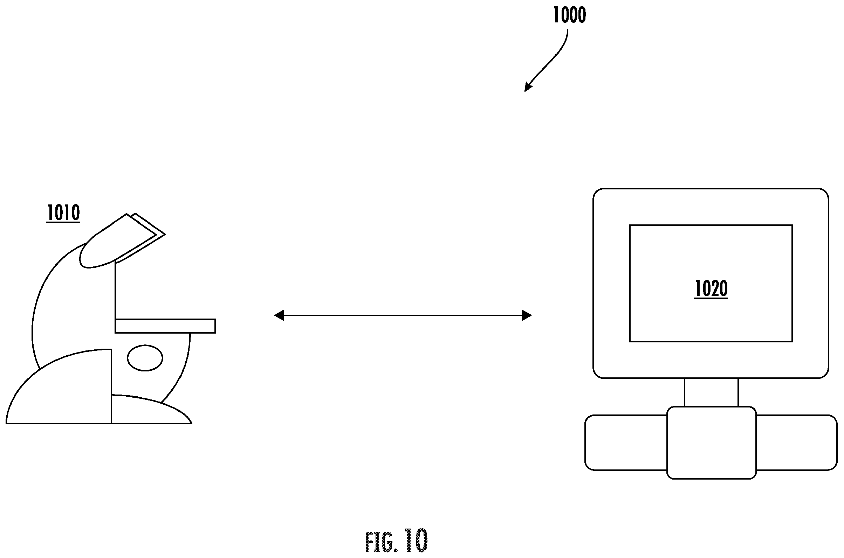

Some embodiments of the present inventive concept relate to a microscope comprising a system as described in connection with one or more of the Figs. 1 to 9. Alternatively, a microscope may be part of or connected to a system as described in connection with one or more of the Figs. 1 to 9. Fig. 10 shows a schematic illustration of a system 1000 configured to perform a method described herein. The system 1000 comprises a microscope 1010 and a computer system 1020. The microscope 1010 is configured to take images and is connected to the computer system 1020. The computer system 1020 is configured to execute at least a part of a method described herein. The computer system 1020 may be configured to execute a machine learning algorithm. The computer system 1020 and microscope 1010 may be separate entities but can also be integrated together in one common housing. The computer system 1020 may be part of a central processing system of the microscope 1010 and/or the computer system 1020 may be part of a subcomponent of the microscope 1010, such as a sensor, an actor, a camera or an illumination unit, etc. of the microscope 1010.

The computer system 1020 may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system 1020 may comprise any circuit or combination of circuits. In one embodiment, the computer system 1-20 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system X20 may be a custom circuit, an application-specific

integrated circuit (ASIC), or the like, such as, for example, one or more circuits (such as a communication circuit) for use in wireless devices like mobile telephones, tablet computers, laptop computers, two-way radios, and similar electronic systems. The computer system 1020 may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system 1020 may also include a display device, one or more speakers, and a keyboard and/or controller, which can include a mouse, trackball, touch screen, voice-recognition device, or any other device that permits a system user to input information into and receive information from the computer system 1020.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the inventive concept can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the inventive concept comprise a data carrier having electronically readable control signals, which are capable of cooperating with a programmable computer system, such that one of the methods described herein is performed.

Generally, embodiments of the present inventive concept can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present inventive concept is, therefore, a computer program having a program code for performing one of the methods described herein, when the computer program runs on a computer.

A further embodiment of the present inventive concept is, therefore, a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present inventive concept is an apparatus as described herein comprising a processor and the storage medium.

A further embodiment of the inventive concept is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the inventive concept comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

The flowchart and block diagrams in the Figures illustrate the architecture, functionality, and operation of possible implementations of systems, methods and computer program products according to various aspects of the present disclosure. In this regard, each block in the flowchart or block diagrams may represent a module, segment, or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

**Claims**

1. A system (930; 1020) for identifying presence of a conjugate in an image comprising one or more processors (938) and one or more storage devices, wherein the system is configured to:

    acquire spectral data associated with a sample to be imaged;
    optimize dispersion coefficients for acquired spectral data;
    calculate a corrective phase function using the optimized dispersion coefficients;
    apply a negative corrective phase function to a signal to provide a resulting image;
    determine if the resulting image has degraded signal strength or an enhanced signal strength relative to an original image;
    calculate a reference arm shift if it is determined that the resulting image has enhanced signal strength; and
    adjust a position of a reference arm of the system based on the calculated reference arm shift to move a conjugate image out of view.

2. The system of Claim 1, wherein the degraded signal strength indicates a non-conjugate signal and an enhanced signal strength indicates a conjugate signal.

3. The system of Claim 1 or 2, wherein the spectral data comprises one of a whole image of the sample, a plurality of A-scans of the sample, data from a plurality of A-scans and sub-regions of the image including a portion of data from a plurality of A-scans .

4. The system of any one of Claims 1-3, wherein the resulting image is an image produced after application of a Fourier transform.

5. The system of any one of Claims 1-4, wherein the system is further configured to adjust the position of the reference arm manually or automatically.

6. The system of any one of Claims 1-5, wherein the system is further configured to calculate the corrective phase function $\phi_c(k)$ using the following equation:

$$\phi_c(k) = c_1(k - k_o)^2 + c_2(k - k_o)^3,$$

where $c_1$ is a first correction coefficient, $c_2$ is a second correction coefficient, $k$ is a source wavenumber and $k_o$ is a center wavenumber of a source.

7. The system of any one of Claims 1-6, wherein the system is configured to adjust the position of the reference arm by providing feedback to the reference arm that causes the reference arm to move a reference reflector to a position where an upright image is in view and a conjugate image is hidden.

8. The system of any one of Claims 1-7, wherein the system includes an optical coherence tomography (OCT) imaging system.

9. The system of any of Claims 1-8, wherein the system further comprises a microscope.

10. A method for identifying presence of a conjugate in an image in a system comprising one or more processors and one or more storage devices, the method comprising:

    acquiring (800) spectral data associated with a sample to be imaged;
    optimizing (810) dispersion coefficients for acquired spectral data;
    calculating (820) a corrective phase function using the optimized dispersion coefficients;
    applying (830) a negative corrective phase function to a signal to provide a resulting image;
    determining (840) if the resulting image has degraded signal strength or an enhanced signal strength relative to an original image;
    calculating (850) a reference arm shift if it is determined that the resulting image has enhanced signal strength; and
    adjusting (860) a position of a reference arm of the system based on the calculated reference arm shift to move a conjugate image out of view.

11. The method of Claim 10, wherein the degraded signal strength indicates a non-conjugate signal and an enhanced signal strength indicates a conjugate signal.

12. The method of Claim 10 or 11, where acquiring spectral comprises acquiring one of a whole image of the sample, a plurality of A-scans of the sample, data from a plurality of A-scans and sub-regions of the image including a portion of data from a plurality of A-scans .

13. The method of Claim 12, wherein the plurality of A-scans comprise less than the entire original image.

14. The method of any one of Claims 10-13, further comprising obtaining the resulting image by applying a Fourier transform.

15. A computer program with a program code for performing the method according to any of Claims 10-14 when the computer program is run on a processor.

**Patentansprüche**

1. System (930; 1020) zur Identifizierung des Vorhandenseins eines Konjugats in einem Bild, das einen oder mehrere Prozessoren (938) und eine oder mehrere Speichervorrichtungen umfasst, wobei das System konfiguriert ist zum:

   Erfassen von Spektraldaten, die einer abzubildenden Probe zugeordnet sind;
   Optimieren von Dispersionskoeffizienten für erfasste Spektraldaten;
   Berechnen einer Korrekturphasenfunktion unter Verwendung der optimierten Dispersionskoeffizienten;
   Anwenden einer negativen Korrekturphasenfunktion auf ein Signal, um ein resultierendes Bild bereitzustellen;
   Bestimmen, ob das resultierende Bild abgeschwächte Signalstärke oder eine verbesserte Signalstärke in Bezug auf ein Originalbild aufweist;
   Berechnen einer Referenzarm-Verschiebung, wenn bestimmt ist, dass das resultierende Bild verbesserte Signalstärke aufweist; und
   Anpassen einer Position eines Referenzarms des Systems basierend auf der berechneten Referenzarm-Verschiebung, um ein Konjugatbild außerhalb der Sicht zu verschieben.

2. System nach Anspruch 1, wobei die abgeschwächte Signalstärke ein Nicht-Konjugatsignal angibt und eine verbesserte Signalstärke ein Konjugatsignal angibt.

3. System nach Anspruch 1 oder 2, wobei die Spektraldaten eines umfassen von einem ganzen Bild der Probe, einer Vielzahl von A-Scans der Probe, Daten aus einer Vielzahl von A-Scans und Teilbereichen des Bilds, die einen Teil von Daten aus einer Vielzahl von A-Scans einschließen .

4. System nach einem der Ansprüche 1-3, wobei das resultierende Bild ein Bild ist, das nach Anwendung einer Fourier-Transformation erzeugt wurde.

5. System nach einem der Ansprüche 1-4, wobei das System weiter so konfiguriert ist, dass es die Position des Referenzarms manuell oder automatisch anpasst.

6. System nach einem der Ansprüche 1-5, wobei das System weiter so konfiguriert ist, dass es die Korrekturphasenfunktion $\phi_c(k)$ unter Verwendung folgender Gleichung berechnet:

$$\phi_c(k) = c_1(k - k_0)^2 + c_2(k - k_0)^3,$$

wobei $c_1$ ein erster Korrekturkoeffizient ist, $c_2$ ein zweiter Korrekturkoeffizient ist, $k$ eine Quellenwellenzahl ist und $k_0$ eine zentrale Wellenzahl einer Quelle ist.

7. System nach einem der Ansprüche 1-6, wobei das System so konfiguriert ist, dass es die Position des Referenzarms durch Bereitstellen von Rückmeldung an den Referenzarm anpasst, die bewirkt, dass der Referenzarm einen Referenzreflektor an eine Position verschiebt, an der ein gerades Bild sichtbar ist und ein Konjugatbild verborgen ist.

8. System nach einem der Ansprüche 1-7, wobei das System ein Bildgebungssystem mit optischer Kohärenztomografie (OCT) einschließt.

9. System nach der Ansprüche 1-8, wobei das System weiter ein Mikroskop umfasst.

10. Verfahren zur Identifizierung des Vorhandenseins eines Konjugats in einem Bild in einem System, das einen oder mehrere Prozessoren und eine oder mehrere Speichervorrichtungen umfasst, wobei das Verfahren Folgendes umfasst:

Erfassen (800) von Spektraldaten, die einer abzubildenden Probe zugeordnet sind;
Optimieren (810) von Dispersionskoeffizienten für erfasste Spektraldaten;
Berechnen (820) einer Korrekturphasenfunktion unter Verwendung der optimierten Dispersionskoeffizienten;
Anwenden (830) einer negativen Korrekturphasenfunktion auf ein Signal, um ein resultierendes Bild bereitzustellen;
Bestimmen (840), ob das resultierende Bild abgeschwächte Signalstärke oder eine verbesserte Signalstärke in Bezug auf ein Originalbild aufweist;
Berechnen (850) einer Referenzarm-Verschiebung, wenn bestimmt ist, dass das resultierende Bild verbesserte Signalstärke aufweist; und
Anpassen (860) einer Position eines Referenzarms des Systems basierend auf der berechneten Referenzarm-Verschiebung, um ein Konjugatbild außerhalb des Sichtbereichs zu verschieben.

11. Verfahren nach Anspruch 10, wobei die abgeschwächte Signalstärke ein Nicht-Konjugatsignal angibt und eine verbesserte Signalstärke ein Konjugatsignal angibt.

12. Verfahren nach Anspruch 10 oder 11, wobei Erfassen von Spektraldaten Erfassen eines von einem ganzen Bild der Probe, einer Vielzahl von A-Scans der Probe, Daten aus einer Vielzahl von A-Scans und Teilbereichen des Bilds umfasst, die einen Teil von Daten aus einer Vielzahl von A-Scans einschließen .

13. Verfahren nach Anspruch 12, wobei die Vielzahl von A-Scans weniger als das gesamte Originalbild umfassen.

14. Verfahren nach einem der Ansprüche 10-13, weiter umfassend Erhalten des resultierenden Bilds durch Anwenden einer Fourier-Transformation.

15. Computerprogramm mit einem Programmcode zum Durchführen des Verfahrens nach einem der Ansprüche 10-14, wenn das Computerprogramm auf einem Prozessor ausgeführt wird.

**Revendications**

1. Système (930 ; 1020) pour identifier la présence d'un conjugué dans une image comprenant un ou plusieurs processeurs (938) et un ou plusieurs dispositifs de stockage, dans lequel le système est configuré pour :

acquérir des données spectrales associées à un échantillon à imager ;
optimiser des coefficients de dispersion pour des données spectrales acquises ;
calculer une fonction de phase corrective en utilisant les coefficients de dispersion optimisés ;
appliquer une fonction de phase corrective négative à un signal pour fournir une image résultante ;
déterminer si l'image résultante présente une puissance de signal dégradée ou une puissance de signal améliorée par rapport à une image d'origine ;
calculer un décalage de bras de référence s'il est déterminé que l'image résultante présente une puissance de signal améliorée ; et
ajuster une position d'un bras de référence du système sur la base du décalage de bras de référence calculé pour déplacer une image conjuguée hors vue.

2. Système selon la revendication 1, dans lequel la puissance de signal dégradée indique un signal non conjugué et une puissance de signal améliorée indique un signal conjugué.

3. Système selon la revendication 1 ou 2, dans lequel les données spectrales comprennent une d'une image entière de l'échantillon, d'une pluralité de balayages axiaux de l'échantillon, de données provenant d'une pluralité de balayages axiaux et de sous-régions de l'image incluant une partie de données provenant d'une pluralité de balayages axiaux.

4. Système selon l'une quelconque des revendications 1 à 3, dans lequel l'image résultante est une image produite après application d'une transformée de Fourier.

5. Système selon l'une quelconque des revendications 1 à 4, dans lequel le système est en outre configuré pour ajuster la position du bras de référence manuellement ou automatiquement.

6. Système selon l'une quelconque des revendications 1 à 5, dans lequel le système est en outre configuré pour calculer la fonction de phase corrective $\phi_c(k)$ en utilisant l'équation suivante :

$$\phi_c(k) = c_1(k - k_0)^2 + c_2(k - k_0)^3,$$

où $c_1$ est un premier coefficient de correction, $c_2$ est un second coefficient de correction, $k$ est un nombre d'onde source et $k_0$ est un nombre d'onde central d'une source.

7. Système selon l'une quelconque des revendications 1 à 6, dans lequel le système est configuré pour ajuster la position du bras de référence en fournissant une rétroaction au bras de référence qui amène le bras de référence à déplacer un réflecteur de référence vers une position où une image droite est en vue et une image conjuguée est cachée.

8. Système selon l'une quelconque des revendications 1 à 7, dans lequel le système inclut un système d'imagerie à tomographie par cohérence optique (OCT).

9. Système selon l'une quelconque des revendications 1 à 8, dans lequel le système comprend en outre un microscope.

10. Procédé pour identifier la présence d'un conjugué dans une image dans un système comprenant un ou plusieurs processeurs et un ou plusieurs dispositifs de stockage, le procédé comprenant :

l'acquisition (800) de données spectrales associées à un échantillon à imager ;
l'optimisation (810) de coefficients de dispersion pour des données spectrales acquises ;
le calcul (820) d'une fonction de phase corrective en utilisant les coefficients de dispersion optimisés ;
l'application (830) d'une fonction de phase corrective négative à un signal pour fournir une image résultante ;
la détermination (840) si l'image résultante présente une puissance de signal dégradée ou une puissance de signal améliorée par rapport à une image d'origine ;
le calcul (850) d'un décalage de bras de référence s'il est déterminé que l'image résultante présente une puissance de signal améliorée ; et
l'ajustement (860) d'une position d'un bras de référence du système sur la base du décalage de bras de référence calculé pour déplacer une image conjuguée hors vue.

11. Procédé selon la revendication 10, dans lequel la puissance de signal dégradée indique un signal non conjugué et une puissance de signal améliorée indique un signal conjugué.

12. Procédé selon la revendication 10 ou 11, dans lequel l'acquisition de données spectrales comprend l'acquisition d'une image entière de l'échantillon, d'une pluralité de balayages axiaux de l'échantillon, de données provenant d'une pluralité de balayages axiaux et de sous-régions de l'image incluant une partie de données provenant d'une pluralité de balayages axiaux.

13. Procédé selon la revendication 12, dans lequel la pluralité de balayages axiaux comprennent moins que la totalité de l'image d'origine.

14. Procédé selon l'une quelconque des revendications 10 à 13, comprenant en outre l'obtention de l'image résultante en appliquant une transformée de Fourier.

15. Programme d'ordinateur avec un code de programme pour réaliser le procédé selon l'une quelconque des revendications 10 à 14 lorsque le programme d'ordinateur est exécuté sur un processeur.

FIG. 1

FIG. 2

FIG. 3

FIG. 4A

DISPERSION
CORRECTION

FIG. 4B

FIG. 5A

FIG. 5B

EP 4 117 504 B1

FIG. 6

FIG. 7

START

ACQUIRE SPECTRAL DATA ASSOCIATED
WITH A SAMPLE TO BE IMAGED — 800

OPTIMIZE DISPERSION COEFFICIENTS — 810

CALCULATE A CORRECTIVE PHASE FUNCTION USING
THE OPTIMIZED DISPERSION COEFFICIENTS — 820

APPLY A NEGATIVE CORRECTIVE PHASE FUNCTION TO
A SIGNAL TO PROVIDE A RESULTING IMAGE — 830

DETERMINE IF THE RESULTING IMAGE HAS DEGRADED
SIGNAL STRENGTH OR ENHANCED SIGNAL STRENGTH — 840

ENHANCED

DEGRADED

CALCULATE A REFERENCE ARM SHIFT IF IT IS
DETERMINED THAT THE RESULTING IMAGE HAS
ENHANCED SIGNAL STRENGTH — 850

ADJUST A POSITION OF THE REFERENCE ARM BASED
ON A THE CALCULATED REFERENCE ARM SHIFT — 860

END

FIG. 8

DISPLAY
988

I/O DATA
PORTS
946

IMAGING
SYSTEM
985

PROCESSOR
938

MEMORY
936

USER
INTERFACE
944

CORRECTIVE
MODULE
987

DATA PROCESSOR
930

FIG. 9

1000

1010

1020

FIG. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62987377
- US 8414564 B
- US 7336366 B2
- US 2016166144 A1

### Non-patent literature cited in the description

- **KIM et al.** *Comparison of phase-shifting techniques for in vivo full-range, high-speed Fourier-domain optical coherence tomography*
- **ZAWADZKI et al.** *AO-OCT with reference arm phase shifting for complex conjugate artifact-free imaging of in vivo retinal structures*